Europäisches Patentamt

European Patent Office

Office européen des brevets

(19)

(11) Publication number: **0 056 825**
**B1**

(12) EUROPEAN PATENT SPECIFICATION

(45) Date of publication of patent specification: **05.12.84**

(21) Application number: **81100468.8**

(22) Date of filing: **22.01.81**

(51) Int. Cl.³: **A 61 K 9/48,** A 61 K 9/52

(54) A process for producing a pharmaceutical capsule having enteric properties.

(43) Date of publication of application:
**04.08.82 Bulletin 82/31**

(45) Publication of the grant of the patent:
**05.12.84 Bulletin 84/49**

(84) Designated Contracting States:
**AT BE CH DE FR GB IT LI NL SE**

(56) References cited:
**EP-A-0 013 566**
**EP-A-0 018 605**
**US-A-3 826 666**
**US-A-3 927 195**
**US-A-4 138 013**

(73) Proprietor: **Capsugel A.G.**
**Engelgasse 11**
**CH-4010 Basel (CH)**

(72) Inventor: **Speiser, Peter P., Prof.Dr.**
**Wasserbergstrasse 26**
**CH-8127 Forch (CH)**
Inventor: **Hersberger, Kurt**
**Andlauerstrasse 8**
**CH-4132 Mutten (CH)**

(74) Representative: **Wuesthoff, Franz, Dr.-Ing. et al**
**Patentanwälte Wuesthoff -v. Pechmann-**
**Behrens-Goetz Schweigerstrasse 2**
**D-8000 München 90 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to pharmaceutical capsules having enteric properties. As used herein, the term "enteric properties" means the properties of being soluble in or disintegrated by the alkaline intestinal secretions but being substantially insoluble or resistant to solution in the acid secretions of the stomach. The term "enteric capsules" means pharmaceutical capsules having enteric properties.

Ordinary pharmaceutical capsules made of gelatin do not have enteric properties and therefore when ingested do not reach the intestine while intact but instead rapidly dissolve or disintegrate in the acid secretions of the stomach. There is, however, a need for pharmaceutical capsules having enteric properties. In many cases, medicinal substances are more readily utilized and are of greater therapeutic value if they are absorbed from the upper portion of the intestine. Medical science has long sought to provide efficient means for rendering such substances available for absorption from the intestinal tract. This problem arises from the fact that many medicinals are either not absorbed from the stomach or rapidly destroyed on contact with the acid present in the stomach. Some examples of medicinals of this type are gland products and penicillin. Many medicinals, such as hog bile, quinacrine, sulfa drugs, and the like, also are very unpleasant to take and cause severe gastric disturbances which may be coupled with very unpleasant regurgitation of the drug. Another use for enteric capsules is to prevent the breakdown or dilution of drugs which are used for their effect in the intestinal tract, such as intestinal antiseptics or anthelmintics. It is obvious that there is a great need for a practical method of administering medicinals of the above types in a form having enteric properties.

Many attempts have been made heretofore to obtain suitable enteric capsules. In general, method of manufacture for enteric capsules fall into one of two categories:

(I) Those comprising treating the exterior of the assembled filled capsules, for example with formaldehyde or glutaraldehyde to decrease the solubility of the capsule wall, or with a coating of an enteric substance; and

(II) Those comprising forming the capsule parts by the dip-moulding technique using a dipping solution which itself after drying possesses enteric properties.

The method according to the present invention falls under category II. Known methods of this type include:

(A) Incorporating in gelatin the alkali or ammonium salts of film-forming agents resistant to gastric juices and conventional production of capsules (Reference: German Patent Application No. 2,336,807 laid open for public inspection; U.S. Patent 4,138,018);

(B) Direct thermoplastic forming into capsules of a mixture of hydroxypropylmethyl-cellulose phthalate and plasticizer Reference: J5—3052619, 1976).

However, it has been found that capsules made according to the mentioned techniques have the following disadvantages: poor solubility in intestinal juice, high organic solvent content, inadequate stability and diffusion problems.

It is an object of the present invention to provide improved enteric capsules.

It is also an object of the invention to provide a process which allows the manufacture of adequately elastic and stable capsules of uniform wall thickness on conventional equipment without organic solvents, without the formation of salts or auxiliary substances resistant to gastric juices, and without thermoplastic forming. It should also be possible to fill the capsules by means of existing equipment, and after-treatment of the filled capsules should not be necessary.

In the present invention, film forming polymers are dispersed in an aqueous solution of a plasticizer. Viscosity-increasing auxiliary substances and anti-foaming agents are added to this dispersion, so as to give an ultrafine, homogeneous dispersion free from air bubbles which is preferably adjusted to a weakly acid pH. By dipping metal pins into this dispersion and subsequently rotating and drying the pins in an air stream of controlled temperature and humidity, capsule halves are obtained which can be stripped from the pins and trimmed to the desired size.

As film-forming polymers the process employs cellulose esters (e.g., cellulose acetate phthalate), cellulose ether esters (e.g. hydroxypropylmethyl-cellulose phthalate), acrylic resins (e.g. Eudragit®) or other natural semi-synthetic or synthetic film-forming substances, in which the particle size should preferably be less than 0.1 mm since as small as possible a particle size facilitates film formation and improves the homogeneity of the film.

The added plasticizers being about good wetting of the polymer and are capable of lowering the film-forming temperature of the polymer. An extremely wide variety of watersoluble or water-miscible plasticizers and mixtures of plasticizers are suitable for this purpose, e.g. glycerol, propylene glycol, mono-acetin, diacetin, triacetin, glycol diacetate, hydroxypropylglycerol and all analogous polyols and the esters or ethers thereof, together with trimethyl citrate, triethyl citrate, di-n-butyl tartrate and other carboxylic acid derivatives. In order to obtain an even film, the plasticizer is preferably in a concentration at which the dispersion does not coagulate. The plasticizer should also have a relatively high boiling point so that there is little plasticizer evaporation and the capsules retain their elasticity and softness.

In the present invention the viscosity of the dispersion is important for the thickness of the capsule wall. The viscosity must be specifically adjusted for each capsule size and for the upper and lower halves of the capsule. For this purpose the process may use highly viscous cellulose ethers such as hydroxypropyl cellulose or hydroxypropylmethyl cellulose.

In order to avoid air inclusions, an anti-foaming agent is used, preferably a silicone anti-foaming emulsion in a concentration of 0.1—2%.

As a dispersion medium the process uses distilled water which is adjusted to a pH at which the gastric juice-resistant film-forming substance is insoluble, e.g. pH 4 to 5. Such a dispersion can readily be stored at room temperature in well-sealed containers, and need only to be homogenized in a non-damaging manner before use.

In the present invention, dye solutions, pigments, antimicrobials, surfactants and other additives may also be included in the dispersions, provided they do not cause the dispersion to coagulate and that film formation is not impaired.

The capsules are produced under conditions of temperature and humidity appropriate to the film-forming temperature of the polymer. In this connection preferably pre-warmed metal pins are dipped into the homogenized dispersion, which is maintained at a temperature of 20°C. The metal pins are then lifted from the dispersion at a uniform speed and the dispersion is allowed to drip from them for a defined period. Drying is then effected with the metal pins rotating at a speed, preferably between 3 and 10 rpm, and with a temperature of 30 to 60°C according to the film-forming temperature and a low relative humidity of less than 30%. During this drying and film-forming process the milky dispersion becomes transparent. As soon as this change is complete, the formed capsule halves can be stripped from the metal pins and trimmed to the correct size.

In order to increase the wall thickness, the dipping process can also be repeated a number of times under the same conditions before the capsules are stripped from the metal pins.

The dipped metal pins may be of a wide variety of shapes, and can be made from any hard metal. In order to facilitate stripping-off the finished capsule halves, the metal pins are slightly greased; this, however, must not impair the wetting of the pins by the dispersion. The process is well suited for all capsule shapes and sizes. Grooves and special shapes such as Snap-Fit®, Coni-Snap® (supplied by Capsugel A.G., Switzerland) and all similar shapes can be produced. Without restricting its scope, the invention is illustrated by the following examples.

Example 1

10.0 g triacetin is dissolved in 170.0 g distilled water. A clear mucilate is prepared by dispersing 1.25 g Ethocel J20MS® (supplied by Dow Chem. Eur.) in this solution and the pH is adjusted to 7/0 with ammonium hydroxide. 40.0 g HP 50%® (supplied by Shinetsu Chem. Jap.) (particle size less than 0.08 mm) is dispersed in this mucilaginous solution.

After the addition of a 2.5 g SE2® anti-foaming emulsion (supplied by Wacker-Chemie, Munich, Germany), this dispersion is left to stand in a closed container for 24 hours at room temperature. The dispersion, which is now free of air bubbles and highly viscous, can be adjusted to the required viscosity with water or with mucilage solution without plasticizer. The pH of this dispersion is between 3 and 5.

Chrome-steel pins pre-warmed to 40°C. (Snap-Fit® capsule size 1, supplied by Capsugel AG, Switzerland) are dipped into this dispersion, which has been homogenized in a non-damaging manner and has a temperature of 20°C. After allowing to drip for 15 seconds, drying is effected by rotation at 4 rpm under controlled temperature and humidity conditions (40°C, 20% rel. hum.). After 60 minutes the film-forming process is completed and a second dipping can take place in accordance with the above description. After a further 90 minutes the capsule halves can be trimmed, stripped from the metal pins and fitted together with the other capsule halves.

Example 2

The same procedure is adopted as described in Example 1, except that 1.60 g Methocel J20MS® is used and 3.5 antifoaming emulsion SE2® is added. The other quantities and auxiliary substances are the same as in Example 1. After dipping, the metal pins are slowly lifted from the dispersion within a period of 5 seconds and immediately rotated at 3 rpm. After drying for approximately 3 hours under the temperature and humidity conditions of Example 1, the capsule halves can be stripped from the metal pins.

Example 3

The same procedure is adopted as described in Example 1, but instead of triacetin a solution of 8.0 triethyl citrate in 172.0 g distilled water is employed.

Example 4

The same procedure is adopted as in Examples 1 and 3, using 1.5 g Klucel Hf® (Hercules BV, Holland) in place of Methocel J20MS®.

Example 5

12.5 g triacetin and 1.25 g Methocel J20MS® are dissolved in 180.0 g distilled water in accordance with Example 1. 40.0 g cellulose acetate phthalate (particle size <0.08 mm) is dissolved in this mucilaginous solution and 5.0 g triacetin is added. After the addition of 2.5 g antifoaming emulsions SE2® the procedure continues as described in Example 1.

The resistance to gastric juice of the capsules made in accordance with the invention was examined as prescribed by Ph.H.VI. The filling employed was methylene blue ground with lactose. In gastric juices of pH 1 at 37°C the filling became spotted with blue after 4 hours, but none of the coloring matter was released. The Snap-Fit® seals the two halves of the capsule sufficiently tightly to prevent gastic juice from entering. During this time the capsules retained their shape and mechanical stability.

In intestinal juice of pH 7.4 at 37°C release begins after a few minutes and the capsule shell has completely dissolved within 15 to 20 minutes.

## Claims

1. A process for producing a pharmaceutical capsule having enteric properties, caracterized by the following steps:

A. Mixing a high viscosity, aqueous dispersion of ultrafine film-forming polymers selected from cellulose esters, cellulose ether esters, acrylic resins, natural, semi-synthetic and synthetic film-forming substances; plasticizers selected from water soluble polyols, their esters and ethers, polycarboxylic acid derivatives and mixtures of such plasticizers; or plasticizers selected from triacetin, citric acid esters and mixture of such plasticizers; viscosity-increasing auxiliary substances selected from highly viscous cellulose ethers; and anti-foaming agents,

B. dipping pre-warmed metal pins into the dispersion and lifting the pins out at a constant speed, the dispersion adhering to the pins being allowed to drip off for a defined length of time and the dispersion subsequently being transformed, during rotation of the metal pins and under controlled temperature and humidity conditions, into a coherent film, and

C. stripping the coherent film from the pins so as to form a wall of the capsule.

2. A process according to claim 1 wherein in step A a coloring agent is added to the aqueous dispersion.

3. A process according to claim 1 wherein in step A an anti-microbial is added to the aqueous dispersion.

4. A process according to claim 1 wherein in step A a surfactant is added to the aqueous dispersion.

5. A process according to claim 1 wherein in Step B the dipping is repeated in order to increase the thickness of the wall of the capsule.

## Revendications

1. Procédé de production d'une capsule pharmaceutique possédant des propriétés entériques, caractérisé par les stades suivants:

A. On mélange une dispersion aqueuse de haute viscosité de polymères filmogènes ultra-fins choisis parmi les esters de cellulose, les esters d'éthers cellulosiques, les résines acryliques, les substances filmogènes naturelles, semi-synthétiques et synthétiques; des plastifiants choisis parmi des polyols solubles, leurs esters et éthers, les dérivés d'acides polycarboxyliques et des mélanges de tels plastifiants; ou des plastifiants choisis parmi la triacétine, les esters d'acide citrique et un mélange de tels plastifiants; des substances auxiliaires d'augmentation de la viscosité choisies parmi les éthers cellulosiques hautement visqueux; et des agents d'antimoussage;

B. On immerge des chevilles métalliques préchauffées dans la dispersion et on enlève les chevilles hors de la dispersion à une vitesse constante, la dispersion adhérante aux chevilles étant autorisée à s'egoutter pendant un laps de temps défini et la dispersion étant ultérieurement transformée, pendant la rotation des chevilles métalliques et dans des conditions réglées de température et d'humidité, en une pellicule cohérente; et

C. On détache la pellicule cohérente des chevilles de manière à former une paroi de la capsule.

2. Procédé selon la revendications 1, dans lequel au stade A, on ajoute un agent colorant à la dispersion aqueuse.

3. Procédé selon la revendication 1, dans lequel au stade A, on ajoute un agent antimicrobien à la dispersion aqueuse.

4. Procédé selon la revendication 1, dans lequel au stade A, on ajoute un surfactif à la dispersion aqueuse.

5. Procédé selon la revendication 1, dans lequel au stade B, on répète l'immersion afin d'augmenter l'épaisseur de la paroi de la capsule.

## Patentansprüche

1. Verfahren zur Herstellung einer pharmazeutischen Kapsel mit enteralen Eigenschaften, gekennzeichnet durch die folgenden Stufen:

A. Vermischen einer hochviskosen wässrighen Dispersion von ultrafeinen filmbildenden Polymeren, ausgewählt aus Celluloseestern, Celluloseether-estern, Acrylharzen, natürlichen, halbsynthetischen und synthetischen film-bildenden Substanzen; Weichmachem, ausgewählt aus wasserlöslichen Polyolen, deren Estern und Ethern, Polycarbonsäurederivaten und Gemischen solcher Weichmacher; oder Weichmachern, ausgewählt aus Triacetin, Citronensäure-estern und Gemischen solcher Weichmacher; die Viskosität erhöhenden Hilfsstoffen, ausgewählt aus hockviskosen Celluloseethern, sowie Antischaummitteln,

B. Eintauchen vorerwärmter Metalldorne in die Dispersion und Herausheben der Dorme mit konstanter Geschwindigkeit, wobei die an den Dornen haftende Dispersion eine definierte Zeit lang abtropfen kann und die Dispersion anschließend während der Rotation der Metalldorne und unter geregelten Temperatur- und Feuchtigkeitsbedingungen zu einem zusammenhängenden Film umgewandelt wird, und

C. Abstreifen des zusammenhängenden Films von den Dornen unter Bildung einer Kapselwand.

2. Verfahren nach Anspruch 1, wobei in Stufe A. ein Färbemittel zu der wässrigen Dispersion zugesetzt wird.

3. Verfahren nach Anspruch 1, wobei in Stufe A. ein antimikrobeielles Mittel zu der wässrigen Dispersion zugesetzt wird.

4. Verfahren nach Anspruch 1, wobei in Stufe A. ein grenzflächechenaktives Mittel zu der wässrigen Dispersion zugesetz wird.

5. Verfahren nach Anspruch 1, wobei in Stufe B. das Eintauchen wiederholt wird, um die Dicke der Kapselwand zu erhöhen.